# EUROPEAN PATENT APPLICATION

(11) **EP 4 803 030 A1**
(43) Date of publication of application: **09.09.2026**
(21) Application number: 25161597.7
(22) Date of filing: 04.03.2025
(51) Int. Cl.: A61B 46/20

(54) **A SURGICAL DRAPE COMPRISING AN ADHESIVE REGION**

(71) Applicant: Mölnlycke Health Care AB, 431 21 Mölndal (SE)
(72) Inventor: OHLSSON, Lisa, 412 65 GÖTEBORG (SE); HAMMARBÄCK-FRIDÉN, Maria, 524 31 HERRLJUNGA (SE)
(74) Representative: Kransell & Wennborg KB

(57) **Abstract**

The present disclosure generally relates to a surgical drape (1) comprising at least one adhesive region (2) configured to adhere to the skin of a patient during use, wherein the at least one adhesive region (2) is arranged along at least a portion of a peripheral edge of the surgical drape (1) or along at least a portion of an internal edge defining an aperture in the surgical drape (1), wherein the adhesive region (2) comprises a first adhesive stripe (3a) arranged adjacent to and substantially parallel to the peripheral edge or the internal edge, and at least a second adhesive stripe (3b) extending substantially parallel to the first adhesive stripe (3a), wherein the first (3a) and second (3b) adhesive stripes are separated by a first non-adhesive region (4).

The present disclosure also relates to a kit comprising the surgical drape (1) and at least one additional component.

## Description

### TECHNICAL FIELD

The present disclosure generally relates to a surgical drape comprising at least one adhesive region configured to adhere to the skin of a patient during use, wherein the adhesive region is arranged along at least a portion of a peripheral edge of the surgical drape or along at least a portion of an internal edge defining an aperture in the surgical drape. The present disclosure also relates to a kit comprising the surgical drape and at least one additional component.

### BACKGROUND

Surgical drapes are essential components in maintaining a sterile field during surgical procedures. The primary purpose of a surgical drape is to isolate the surgical site from other areas of the patient's body and from non-sterile surfaces of the surroundings, such as surgery tables, in order to reduce the risk of surgical site infection.

The process of applying a surgical drape onto a patient prior to surgery is referred to as draping. Draping a patient is typically a difficult and cumbersome task for the surgical staff. A surgical drape is extremely large and bulky, and therefore difficult to handle.

In many surgical procedures, a plurality of surgical drapes is used to drape a patient. For example, multiple drapes may be applied to the skin of a patient to define an aperture, i.e. an exposed skin area, where the surgery is to be performed.

Typically, each surgical drape comprises a wide adhesive layer along one (or more than one) peripheral edge of the drape. The adhesive layer of each drape is attached to the skin in the area surrounding the aperture to create a sealed barrier.

However, during application (i.e. draping), folds and wrinkles may inadvertently form in the adhesive area surrounding the aperture. This may give rise to the formation of liquid channels, which allows wound exudate to leak from the surgical site. Such leakage compromises the sterile barrier and increases the risk of contamination. Furthermore, the risk of lifting or detachment of the drape during surgery is enhanced, which further reduces the effectiveness of the protective seal.

While proper adhesion and sealing at the surgical site are critical, this must also be carefully balanced against the risk of causing skin trauma upon removal. Strong adhesives covering a large and wide surface area may cause skin irritation, redness, and sometimes stripping of the outer skin layers, particularly in patients with sensitive or fragile skin.

Furthermore, the medical industry is shifting towards more sustainable products to reduce waste and environmental impact. The use of excessive amounts of adhesive may increase material consumption and the environmental burden.

In view of the above-mentioned challenges, there is a need for a sustainable and improved surgical drape, which enhances the sterility during draping and during surgery, facilitates the draping procedure for the surgical staff, and which minimizes the risk of causing skin trauma upon removal.

### SUMMARY

In view of the above-mentioned problems, it is an object of the present disclosure to provide improvements in the field of surgical drapes by reducing the environmental impact, improving the sterility and the patient comfort.

According to a first aspect, there is provided a surgical drape comprising at least one adhesive region configured to adhere to the skin of a patient during use, wherein the adhesive region is arranged along at least a portion of a peripheral edge of the surgical drape or along at least a portion of an internal edge defining an aperture in the surgical drape, wherein the adhesive region comprises a first adhesive stripe arranged adjacent to and substantially parallel to the peripheral edge or the internal edge, and at least a second adhesive stripe extending substantially parallel to the first adhesive stripe, wherein the first and second adhesive stripes are separated by a first non-adhesive region.

The surgical drape of the present disclosure provides several advantages over conventional surgical drapes. For example, if wrinkles or folds form along the peripheral edge of the surgical drape, i.e. in the area of the first adhesive stripe, the second adhesive stripe serves as a "backup" and prevents potential liquid channels formed in the first adhesive stripe from reaching further. An improved seal against contamination is thus provided.

Furthermore, the presence of a non-adhesive region between the stripes reduces the overall adhesive contact area with the skin, which may help to minimize irritation and the risk of causing skin damage upon removal.

By dividing the adhesive into several discrete stripes, the total amount of adhesive material is reduced, which may contribute to a more sustainable configuration with lower material consumption.

In exemplary embodiments, the adhesive region further comprises a third adhesive stripe arranged substantially parallel to the first and second adhesive stripes, wherein the second and third adhesive stripes are separated by a second non-adhesive region.

The provision of a third adhesive stripe (and a second non-adhesive region) provides an additional level of protection in cases where folds or wrinkles form during draping. In the event of liquid channels formed during draping, the provision of a third adhesive stripe reduces the risk of fluid leakage reaching beyond the adhesive region, thereby improving the overall integrity of the sterile barrier.

The adhesion to the skin of a patient is also enhanced, however, the reduced total adhesive contact area (compared to using a wide single adhesive layer) prevents skin irritation and trauma upon removal.

In exemplary embodiments, the first adhesive stripe has a width, w1, and the second adhesive stripe has a width, w2, wherein each of the widths, w1 and w2, is from 4 to 15 mm, preferably from 6 to 12 mm.

A width in this range secures an optimal balance between secure attachment to the skin and patient comfort.

The widths, w1, and w2, are typically substantially the same. However, it is also conceivable that w1 and w2 may differ. For example, in some embodiments, w2 is less than w1.

In embodiments where the adhesive region comprises three adhesive stripes, the third adhesive stripe may have a width, w3, of from 4 to 15 mm, preferably from 6 to 12 mm.

In exemplary embodiments, the first non-adhesive region may have a width, w4, of from 3 to 20 mm, preferably from 4 to 15 mm.

If the width, w4, of the first non-adhesive region is too large, this may yield insufficient sealing. In contrast, if the width is too narrow, the adhesion to the skin may be too firm such that skin irritation or trauma occurs upon removal of the surgical drape. The above-mentioned range ensures an optimal balance between secure attachment, flexibility and patient comfort.

In embodiments where the adhesive region comprises three adhesive stripes, the second non-adhesive region may have a width, w5, of from 3 to 20 mm, preferably from 4 to 15 mm.

The widths, w4, and w5, are typically substantially the same. However, it is also conceivable that w4 and w5 differ depending on the type of drape and type of surgical procedure to be performed.

Surgical drapes are available in various configurations. For example, surgical drapes referred to as "aperture drapes" have a predefined aperture that exposes a surgical site during use. So called "universal drapes" are drapes that can be applied to different parts of the body and be adapted for various surgical procedures. These drapes typically do not have a pre-cut aperture but can be arranged, i.e. combined with additional universal drapes, to create an aperture, as needed. Another type of drape is a "split drape", which comprises a split or channel that allows it to be wrapped around a limb or another anatomical structure.

For each of these surgical drapes, a secure and sterile seal around a surgical site must be provided.

In exemplary embodiments, the surgical drape may comprise an aperture; the aperture being defined by internal edges, and wherein the adhesive region extends continuously along each of these internal edges.

Accordingly, the drape may be an aperture drape. The adhesive region extends continuously along the entire perimeter of the aperture, which significantly reduces the risk of gaps, and localized detachment or folding around the aperture/surgical site. Furthermore, in surgical procedures involving high levels of exudate, such as laparoscopic or orthopedic surgeries, wound exudate is prevented from leaking beyond the sterile field.

In exemplary embodiments, the surgical drape has a lateral (x) and a longitudinal (y) extension and is defined by a first and second peripheral edge along the longitudinal extension and a third and fourth peripheral edge along the lateral extension, wherein the adhesive region may be arranged adjacent to and along at least a portion of the third and/or fourth peripheral edge.

Accordingly, the surgical drape may be a universal drape, i.e. a drape which may be adapted for various surgical procedures (rather than being pre-configured for a specific application).

Typically, a plurality of universal drapes is utilized in combination to create a customized sterile field around the surgical site. In such scenarios, each lateral edge provided with an adhesive region of each surgical drape contributes to forming a closed seal around the surgical site. By overlapping or aligning the adhesive regions of adjacent drapes, a continuous seal is established around a surgical site, which minimizes the risk of fluid leakage and microbial contamination.

In exemplary embodiments, the at least one adhesive region is arranged adjacent to and along at least 30% of the length of the third and/or fourth peripheral edge.

The extension of the adhesive region along the lateral edge may vary depending on the type of surgical procedure to be performed. In some procedures (and for some surgical drapes), it may be beneficial to cover the entire lateral edge. However, for many surgical procedures, it is only required to have the adhesive region in the area where adhesion to the skin is actually required, i.e. to form an aperture around a surgical site.

Accordingly, the extension of the adhesive region may vary along the length of the third and/or fourth lateral edge but is typically at least 30% of its length. This way, the adhesive is applied only where needed such that the amount of adhesive material may be reduced. Furthermore, unnecessary adhesion to the patient's skin is avoided (which may also facilitate repositioning during draping).

In exemplary embodiments, the at least one adhesive region is arranged adjacent to and along 30-80% of the length of the third and/or fourth peripheral edge, and wherein the adhesive region is centrally disposed along the length of the third and/or fourth peripheral edge.

Centrally positioning the adhesive region ensures efficient sealing, and also allows for flexibility at the outer ends of the lateral (third or fourth) edge.

As mentioned hereinbefore, for some procedures, it may be advantageous to have adhesive along the entire length of the third or fourth peripheral edge to ensure full-seal coverage. However, for many applications, partial adhesive coverage is sufficient, preventing the use of excess adhesive where it is not necessary. By optimizing adhesive placement, this configuration reduces material waste, lowers costs, and maintains sterility during surgery.

In exemplary embodiments, the at least one adhesive edge portion may comprise a pressure-sensitive adhesive (PSA).

Hence, the adhesive region adheres to the skin upon light pressure. The pressure-sensitive adhesive provides strong adhesion, while also ensuring a gentle removal from the skin.

The pressure-sensitive adhesive may be a polyacrylate-based adhesive, a silicone-based adhesive, or a rubber-based adhesive.

In exemplary embodiments, each of the first adhesive stripe, the second adhesive stripe, and the third adhesive stripe, if present, may have a coating weight of from 25 to 35g/m2.

Accordingly, the total amount of adhesive used is significantly lower compared to a traditional wide adhesive layer. Furthermore, a coating weight in the above-mentioned range ensures sufficient adhesion strength while minimizing skin irritation and discomfort upon removal.

In exemplary embodiments, the at least one adhesive region may be covered by a release liner prior to use.

The release liner protects the adhesive region before use and prevents it from becoming contaminated.

According to another aspect, there is provided a kit comprising the surgical drape as described hereinbefore and at least one additional component.

The kit may comprise additional components needed during surgery. For example, the kit may comprise all the drapes and components needed for a specific surgical procedure. Hence, one kit may contain all components (e.g. surgical drapes, syringes, scissors, trocars, tubings, gauzes, surgical gowns) required for a specific surgery, e.g. a laparoscopic surgical procedure, a specific orthopedic surgical procedure etc.

In exemplary embodiments, the kit comprises at least four surgical drapes as described hereinbefore.

The four surgical drapes are preferably four universal surgical drapes. Hence, these drapes may be assembled on-site and arranged such that the adhesive region of the respective drape forms an aperture, i.e. a sterile opening around the surgical site.

The surgical drapes (and additional kit components) may be provided in a prepackaged kit. This is advantageous as it facilitates the surgical preparation process for the surgical staff. All necessary components needed for draping are thus readily available, which reduces the setup time and contributes to a more streamlined workflow for the surgical staff.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 schematically illustrates a surgical drape according to an exemplary embodiment of the present disclosure.
Figure 2 schematically illustrates an opening formed by one or multiple surgical drapes according to exemplary embodiments of the present disclosure. The opening exposes the skin area where a surgical intervention is to be performed.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

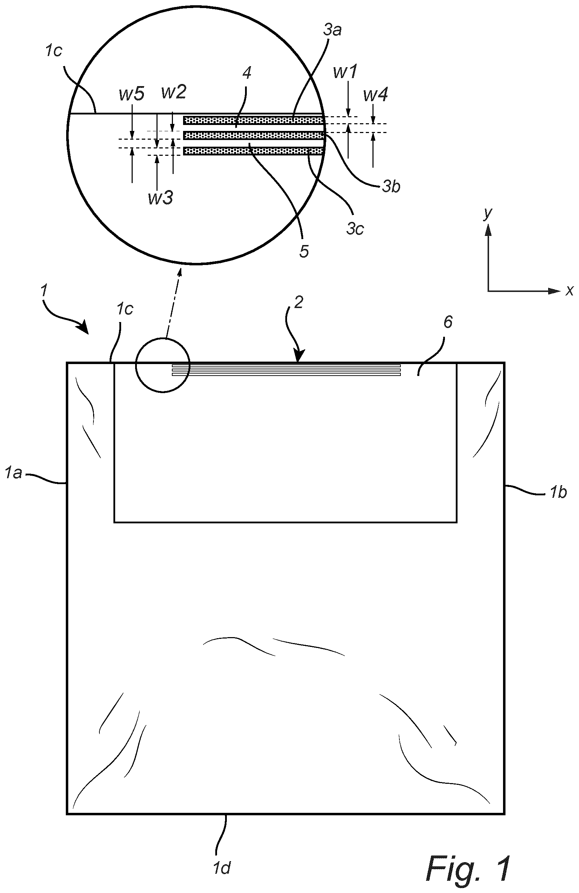

A surgical drape is schematically illustrated in figure 1. The surgical drape 1 comprises at least one adhesive region 2 configured to adhere to the skin of a patient during use, wherein the adhesive region 2 is arranged along at least a portion of a peripheral edge 1a-d of the surgical drape 1 or along at least a portion of an internal edge defining an aperture in the surgical drape 1, wherein the adhesive region 2 comprises a first adhesive stripe 3a arranged adjacent to and substantially parallel to the peripheral edge or the internal edge, and at least a second adhesive stripe 3b extending substantially parallel to the first adhesive stripe 3a, wherein the first adhesive stripe 3a and the second adhesive stripe 3b are separated by a first non-adhesive region 4.

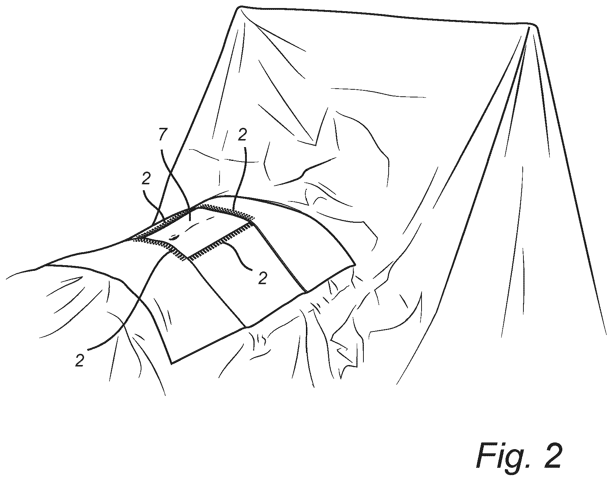

As used herein, the term "surgical drape" means a sterile sheet or cover for use in medical procedures to create a barrier between the surgical field and non-sterile areas to reduce the risk of contamination. The surgical drape may comprise a nonwoven and/or a woven material. As illustrated in figure 2, the surgical drape may comprise areas which are more absorbent than others (see area denoted 6 in figure 2). In the context of the present disclosure, the surgical drape is a drape that is configured to be at least partially adhered to the skin of a patient during use. The surgical drape may be a universal drape, an aperture drape or a split drape.

The size and shape of the surgical drape 1 may vary depending on the surgical procedure to be performed. The surgical drape 1 may comprise one sheet. Alternatively, the surgical drape 1 may comprise several sheets or panels.

The surgical drape 1 comprises at least one adhesive region 2.

The surgical drape 1 has a top side and a bottom side. The adhesive region 2 is arranged on the bottom side of the surgical drape 1.

The adhesive region 2 comprises a first adhesive stripe 3a and at least a second adhesive stripe 3b. The first adhesive stripe 3a is arranged adjacent to and substantially parallel to the peripheral edge or the internal edge of the surgical drape.

That the first adhesive stripe "is arranged adjacent to" the peripheral edge or the internal edge means that the first adhesive stripe 3a is positioned in close proximity to the peripheral edge or the internal edge of the surgical drape. Hence, the first adhesive stripe 3a extends along and in proximity of the peripheral edge or internal edge without necessarily being in direct contact with the edge. For example, the first adhesive stripe 3a may be arranged at a distance of from 1 to 8 mm, e.g. from 1 to 5 mm from the peripheral edge or internal edge. The first adhesive stripe 3a is preferably arranged as close as possible to the peripheral or internal edge.

That the "first adhesive stripe is arranged substantially parallel to the peripheral edge" means that the first adhesive stripe extends in the same general direction as the peripheral edge or internal edge of the surgical drape. While minor variations in alignment may occur due to e.g. manufacturing tolerances, the adhesive stripe is predominantly parallel to the edge.

The second adhesive stripe 3b is arranged substantially parallel to the first adhesive stripe 3a.

As best illustrated in the zoomed-in view of figure 1, the first adhesive stripe 3a and the second adhesive stripe 3b are separated by a first non-adhesive region 4.

As shown in figure 1, the adhesive region 2 may further comprise a third adhesive stripe 3c. The third adhesive stripe 3c is arranged substantially parallel to the second adhesive stripe 3b (and substantially parallel to the first adhesive stripe 3a).

Hence, the second adhesive stripe 3b may be arranged between the first 3a and the third 3c adhesive stripes.

The second 3b and third 3c adhesive stripes are separated by a second non-adhesive region 5.

In the adhesive region 2, each of the adhesive stripes 3a-c typically extend continuously without interruption. Hence, the first non-adhesive region 4, and the second non-adhesive region 5 define non-adhesive channels between the adhesive stripes 3a-c. The non-adhesive region(s) prevents the surgical drape from adhering too hard to the skin and prevents skin irritation and trauma during removal from the skin.

The length of each adhesive stripe 3a-c may vary depending on the shape and size of the drape, as well as the surgical procedure to be performed. The length of the adhesive stripes (and the adhesive region) may vary depending on how large opening or aperture that is to be formed on the patient during the surgical procedure.

The length of the first 3a and second 3b adhesive stripes is typically the same. The length of the third adhesive stripe 3c, where present, is the same as the length of the first 3a and second 3b adhesive stripes.

The length of the first 3a, second 3b, and third 3c adhesive stripe (if present) may be from 150 to 1500 mm, e.g. from 300 to 1000 mm.

The width of the first 3a and second 3b adhesive stripes may be the same or it may differ. The width of the third adhesive stripe 3c, where present, may be the same as the width of the first 3a and/or second 3b adhesive stripes or it may be different. Typically, the adhesive stripes 3a-c have the same or at least substantially the same widths.

For example, the first adhesive stripe 3a has a width, w1; the second adhesive stripe 3b has a width, w2, and the third adhesive stripe 3c (if present) has a width, w3, wherein each of the widths, w1, w2, and/or w3 may be from 4 to 15 mm, preferably from 6 to 12 mm.

The first non-adhesive region 4 is arranged between the first 3a and second 3b adhesive stripes. The second non-adhesive region 5 (if present) is arranged between the second 3b and third 3c adhesive stripes.

The length of the non-adhesive region(s) (4,5) is the same as the length of each adhesive stripe 3a-c.

The width of the first non-adhesive region 4 may be the same as the width of the second non-adhesive region 5 or it may be different. Typically, the width is the same.

For example, the first non-adhesive region 4 may have a width, w4, of from 3 to 20 mm, preferably from 4 to 15 mm.

The second non-adhesive region 5 may have a width, w5, of from 3 to 20 mm, preferably from 4 to 15 mm.

Each of the first adhesive stripe, the second adhesive stripe, and the third adhesive stripe, if present, may have a coating weight of from 25 to 35g/m2.

As used herein, the term "coating weight" refers to the amount of adhesive applied per unit area of the substrate (the surgical drape). The coating weight provides an indication of the adhesive distribution and thickness.

A coating weight in the above-mentioned range is beneficial to reduce the total amount of adhesive used in the adhesive region, while still enabling tight adhesion to the skin. It also minimizes skin irritation and discomfort upon removal.

The thickness of the first adhesive stripe 3a, the second adhesive stripe 3b, and third adhesive stripe 3c, if present, may be from 20 µm to 200 µm, preferably from 40 µm to 100 µm.

The surgical drape may be an aperture drape, i.e. a drape comprising a predefined aperture.

The aperture is defined by internal edges, and the adhesive region 2 may be arranged to extend continuously along each of these internal edges.

As used herein, the term "aperture" refers to an opening in the surgical drape that exposes a portion of the patient's skin, defining the surgical site. The aperture is not limited to a specific shape, but any shape is conceivable. Typically, the aperture is square shaped or rectangular. It is also conceivable that the aperture is circular or elliptical. The internal edges of the aperture provide the interface between the drape and the exposed skin. Proper adhesion at the internal edges is important to prevent detachment, folds, fluid leakage, and/or microbial contamination.

The internal edges may be straight or curved.

The adhesive region 2 extends continuously along each internal edge, which improves the sterile barrier, and reduces the risk for localized detachment.

The aperture drape may have the general configuration as illustrated in figure 2. In this figure, the adhesive region 2 is shown around the aperture 7. This is just for illustrative purposes, since the adhesive region is typically not visible from the top side of the surgical drape (it is arranged on the bottom side of the surgical drape).

It is also conceivable that the aperture 7 in figure 2 is formed by arranging multiple surgical drapes (typically four different drapes) onto the skin of a patient. In such embodiments, at least one peripheral edge of the surgical drape comprises the adhesive region 2. As mentioned hereinbefore, these types of surgical drapes may be referred to as universal drapes. Instead of containing a pre-cut aperture, these surgical drapes form an aperture when combined and assembled on site.

The universal drape may have the general configuration as illustrated in figure 1.

As illustrated in figure 1, the surgical drape 1 has a lateral (x) and a longitudinal (y) extension and is defined by a first 1a and a second 1b peripheral edge along the longitudinal (y) extension, and a third 1c and a fourth 1d peripheral edge along the lateral (x) extension, wherein at least one adhesive region 2 is arranged adjacent to and along at least a portion of the third 1c and/or fourth 1d peripheral edge.

This configuration allows the surgical staff to adapt the sterile field, i.e. create a suitable sized aperture depending on the surgical procedure to be performed.

The first 1a and second 1b peripheral edges may be referred to as longitudinal edges.

The third 1c and fourth 1d may be referred to as lateral edges.

The surgical drape 1 illustrated in figure 1 has a rectangular shape. In other words, the longitudinal (y) extension of the surgical drape is larger than the lateral (x) extension of the surgical drape.

It should be noted that the surgical drape is not limited to a specific shape, but any shape is conceivable. Furthermore, the peripheral edges 1a-d need not be straight, but may be curved, angled or irregularly shaped.

In figure 1, the adhesive region 2 is arranged adjacent to and along at least a portion of the third peripheral edge 1c of the surgical drape.

The at least one adhesive region is arranged adjacent to and along at least 30% of the length of the third and/or fourth peripheral edge.

The length of the third or fourth peripheral edges may also be referred to as the maximum lateral (x) extension of the third or fourth peripheral edges. The length of the lateral edges may vary depending on e.g. the surgical procedure to be performed and the size of the patient.

For example, the length of the third and fourth lateral peripheral edges may be from 50 to 250 cm, e.g. from 80 to 180 cm.

Likewise, the length of the first and/or fourth peripheral edges may vary depending on e.g. the surgical procedure to be performed and the size of the patient. The length of the first or second peripheral edges may also be referred to as the maximum longitudinal (y) extension of the first or second peripheral edges

For example, the length of the first or second longitudinal peripheral edges may be from 150 to 350 cm, e.g. from 150 to 250 cm.

The lengths, discussed hereinbefore, refer to the maximum length (i.e., the maximum longitudinal (y) extension). If the surgical drape has an irregular shape, the maximum length should be interpreted as the longest measurable distance between the first and second longitudinal edges, irrespective of curved, angled, or non-linear edge configurations.

As illustrated in figure 1, the adhesive region 2 is arranged adjacent to and along 30-80% of the length of the third and/or fourth peripheral edge, and wherein the adhesive region 2 is centrally disposed along the length of the third peripheral edge 1c.

In figure 1, the adhesive region 2is arranged along about 50% of the length of the third peripheral edge 1c.

The extent of "adhesive coverage" along the length of the third and/or fourth peripheral edge may vary depending on the specific surgical procedure, the required sterile field, and the patient size.

In some embodiments, the adhesive region 2 is arranged adjacent to and along 100% of the length of the third and/or fourth peripheral edge.

The at least one adhesive edge region 2 may comprise a pressure-sensitive adhesive (PSA).

As used herein, the term "pressure-sensitive adhesive" refers to an adhesive that adheres to a surface, such as the skin, upon the application of light pressure (without requiring heat, water, or solvents for activation).

For example, the pressure-sensitive adhesive may be a polyacrylate-based adhesive, a silicone-based adhesive, or a rubber-based adhesive. The selection of PSA may vary depending on the type of surgical drape, the required adhesion strength, and specific procedural requirements. Typically, the pressure-sensitive adhesive is a polyacrylate adhesive.

Prior to use, the at least one adhesive region 2 is covered by at least one release liner (not shown).

The release liner protects the adhesive region 2 from contamination and unintentional adhesion before application. The size and shape of the release liner typically corresponds to, or is larger than, the size and shape of the adhesive region. Hence, the release liner is arranged to cover each adhesive stripe of the adhesive region.

According to another aspect, there is provided a kit comprising the surgical drape as described hereinbefore and at least one additional component.

The additional component may be at least one additional surgical drape and/or a component selected from a trocar, syringe, scissor, scalpel, tubing, gauze, surgical gown or surgical gloves.

The kit may comprise all components needed for a specific surgical procedure, e.g. a laparoscopic or an orthopedic surgical procedure.

The provision of the necessary components in a single kit facilitates efficient surgical preparation, reduces setup time, and secures that the surgical staff has direct access to all components needed, which streamlines the workflow and minimizes errors.

\In embodiments where universal drapes are to be used, the kit typically comprises at least four surgical drapes as described hereinbefore. For these drapes, the adhesive region 2 is arranged along a peripheral edge of the surgical drape.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A surgical drape (1) comprising at least one adhesive region (2) configured to adhere to the skin of a patient during use, wherein said at least one adhesive region (2) is arranged along at least a portion of a peripheral edge (1a-d) of said surgical drape (1) or along at least a portion of an internal edge defining an aperture in said surgical drape (1), **characterized in that** said adhesive region (2) comprises a first adhesive stripe (3a) arranged adjacent to and substantially parallel to said peripheral edge (1a-d) or said internal edge, and at least a second adhesive stripe (3b) extending substantially parallel to said first adhesive stripe (3a), wherein said first (3a) and second (3b) adhesive stripes are separated by a first non-adhesive region (4).

2. The surgical drape (1) according to claim 1, wherein said adhesive region (2) further comprises a third adhesive stripe (3c) arranged substantially parallel to said first (3a) and second (3b) adhesive stripes, wherein said second (3b) and third (3c) adhesive stripes are separated by a second non-adhesive region (5).

3. The surgical drape (1) according to claim 1 or claim 2, wherein said first adhesive stripe (3a) has a width, w1; said second adhesive stripe (3b) having a width, w2, wherein each of said widths, w1 and w2, is from 4 to 15 mm, preferably from 6 to 12 mm.

4. The surgical drape (1) according to claim 2 or claim 3, wherein said third adhesive stripe (3c) has a width, w3, of from 4 to 15 mm, preferably from 6 to 12 mm.

5. The surgical drape (1) according to any one of claims 1-4, wherein said first non-adhesive region (4), has a width, w4, of from 3 to 20 mm, preferably from 4 to 15 mm.

6. The surgical drape (1) according to any one of claims 2-5, wherein said second non-adhesive region (5), has a width, w5, of from 3 to 20 mm, preferably from 4 to 15 mm.

7. The surgical drape (1) according to any one of claims 1-6, wherein said surgical drape comprises an aperture (7); said aperture (7) being defined by internal edges, and wherein said adhesive region (2) is arranged to extend continuously along each of said internal edges.

8. The surgical drape (1) according to any one claims 1-7, wherein said surgical drape (1) has a lateral (x) and a longitudinal (y) extension and is defined by a first (1a) and a second (1b) peripheral edge along the longitudinal (y) extension of said surgical drape (1), and a third (1c) and a fourth (1d) peripheral edge along the lateral (x) extension of said surgical drape, wherein said at least one adhesive region (2) is arranged adjacent to and along at least a portion of said third (1c) and/or fourth (1d) peripheral edge.

9. The surgical drape (1) according to claim 8, wherein said at least one adhesive region (2) is arranged adjacent to and along at least 30% of the length of said third (1c) and/or fourth peripheral edge (1d).

10. The surgical drape (1) according to claim 9, wherein said at least one adhesive region (2) is arranged adjacent to and along 30-80% of the length of said third (1c) and/or fourth (1d) peripheral edge, and wherein said adhesive region (2) is centrally disposed along said third (1c) and/or fourth (1d) peripheral edge.

11. The surgical drape (1) according to any one of claims 1-10, wherein said at least one adhesive region (2) comprises a pressure-sensitive adhesive.

12. The surgical drape (1) according to any one of claims 1-11, wherein each of said first adhesive stripe (3a), said second adhesive stripe (3b), and said third adhesive stripe (3c), if present, has a coating weight of from 25 to 35 g/m2.

13. The surgical drape (1) according to any one of claims 1-12, wherein said at least one adhesive region (2) is covered by at least one release liner prior to use.

14. A kit comprising the surgical drape (1) according to any one of claims 1-13 and at least one additional component.

15. The kit according to claim 14 comprising at least four surgical drapes according to any one of the preceding claims.
